# EUROPEAN PATENT SPECIFICATION

(11) **EP 4 255 546 B1**
(45) Date of publication and mention of the grant of the patent: **06.03.2024**
(21) Application number: 21863082.0
(22) Date of filing: 01.12.2021
(51) Int. Cl.: A61M 25/00, A61M 27/00

(54) **PACKAGED CATHETER ASSEMBLY**
VERPACKTE KATHETERANORDNUNG
ENSEMBLE CATHÉTER EMBALLÉ

(30) Priority: 04.12.2020 GB 202019180; 17.12.2020 GB 202019944
(43) Date of publication of application: 11.10.2023
(73) Proprietor: ConvaTec Limited, Deeside, Flintshire CH5 2NU (GB)
(72) Inventor: PFLEGER, Oliver W., Flintshire CH5 2NU (GB); MECIAR, Branislav, 071 01 Michalovce (SK); NOVAK, Marian, 071 01 Michalovce (SK)
(74) Representative: Wilson Gunn
(86) International application number: PCT/GB2021/053129
(87) International publication number: WO 2022/118011

(56) References cited:
- EP-A1- 3 095 724
- CN-U- 211 382 187
- GB-A- 2 371 036
- US-A- 5 131 537
- US-A1- 2004 074 794

## Description

### Technical Field of the Invention

The present invention relates to packaged catheter assemblies. In particular to packaged urinary catheter assemblies, and most particularly to packaged intermittent male urinary catheter assemblies.

### Background to the Invention

Many people suffer from urinary problems that can make it difficult to pass urine. Intermittent catheter assemblies can provide a convenient and portable solution to this problem as a user is able to self-catheterise to relieve themselves as required. This reduces the effect of their urinary condition on their life and allows them to enjoy a relatively normal lifestyle.

Intermittent catheters are typically single-use devices that are carried by the user then unpackaged and used as required. To reduce the risk of damage to the urinary tract and improve comfort while using the catheter, it is important they are lubricated before use. However, lubricating a catheter in a public place, for example a public bathroom, can be difficult and inconvenient. This increases the risk that he catheter is not suitably lubricated prior to use which can result in discomfort and injury.

Male intermittent catheters are generally several centimetres longer than female catheters, typically at least 20cm long, with ISO 20696:2018 setting a minimal effective length of 275mm. This can make them difficult to carry in a discreet manner. The additional length can also make them more difficult to adequately lubricate and use.

EP3095724A1 discloses a catheter assembly comprising a catheter and a package with a compartment for accommodating the catheter. The package comprises a non-transparent part and a transparent part, the transparent part allowing visual identification of the orientation and/or state of the catheter in the package.

US2004/0074794A1 discloses a system and method for wetting a catheter. The system includes a catheter container, a catheter and a fluid packet. The catheter container can be formed from a sheet of transparent material and a sheet of opaque or translucent material to provide a view of the catheter inside the container.

US5131537A discloses packaging for a catheter. The packaging includes an elongate pouch or overwrap and a mounting card, on which the catheter is supported. The elongate pouch comprises two flexible sheet members. One sheet is made from a generally opaque material and the other sheet is generally transparent.

GB2371036A discloses a package for a medical tube comprising front and back panels and a fluid reservoir and at least part of the package is transparent to allow viewing and control of the lubrication of the catheter.

It is an object of embodiments of the present invention to at least partially overcome or alleviate the above problems.

### Summary of the Invention

According to a first aspect of the invention there is provided a packaged catheter assembly according to claim 1.

According to a second aspect of the invention there is provided a method of forming a packaged catheter assembly according to claim 12.

Thus, the present invention provides for a packaged catheter assembly that is discreet as the contents of the assembly are hidden. The window provides a visual guide so that during use of the catheter, the user can easily identify the fluid reservoir, and/or easily identify that fluid has been successfully released from the reservoir into a wetting region of the pouch to lubricate the catheter prior to use. Whilst the provision of a transparent/translucent window is the preferred approach for allowing a user to view whether fluid has been successfully released from the reservoir, alternative approaches are considered feasible. For example, rather than providing a transparent/translucent window in an opaque wall of the pouch, it is conceived that the functionality could be achieved by providing a transparent/translucent pouch wall, but including an intervening opaque wall, within the pouch provided between the transparent/translucent pouch wall and the catheter (and optionally the reservoir). The intervening wall could include a window, or fluid released from the reservoir could flow between the outer transparent/translucent wall and the intervening wall, providing an indication of successful release.

The following optional/preferred features apply to the invention in general terms, including both the first and second aspects outlined above, as well as to the further aspects set out below.

In the catheter assembly of the first aspect, in some embodiments the catheter is arranged within the pouch in a curved configuration; and a join is provided between walls of the pouch, so as to define a channel into which fluid can be introduced before withdrawal of the catheter from the pouch so that during withdrawal of the catheter from the pouch, (with the distal end of the catheter withdrawn from the pouch before the proximal end, e.g. by pulling out by the distal end) the proximal end of the catheter passes through the channel.

In the catheter assembly of the first aspect, in some embodiments the pouch comprises two walls and a peripheral seal between the two walls; the catheter comprises a proximal end for insertion into the body and a distal end; the catheter is arranged within the pouch in a curved configuration; and a join is provided between the two walls, wherein: the join and an internal base of the peripheral seal define a channel to be filled with fluid before withdrawal of the catheter from the pouch; and the pouch and catheter are arranged such that during withdrawal of the catheter from the pouch, the distal end of the catheter is withdrawn from the pouch before the proximal end and the proximal end of the catheter passes through the channel.

The method of the second aspect may comprise: arranging the catheter inside the pouch in a curved configuration; and forming a join between walls of the pouch to define a channel into which fluid can be introduced before withdrawal of the catheter from the pouch; so that during withdrawal from the pouch (with the distal end of the catheter withdrawn from the pouch before the proximal end, e.g. by pulling out by the distal end) the proximal end of the catheter passes through the channel.

The method of the second aspect may comprise: providing two walls to define a pouch; arranging the catheter inside the pouch in a curved configuration; and forming a join between the walls, wherein: the join and an internal base of the peripheral seal define a channel to be filled with fluid before withdrawal of the catheter from the pouch; the catheter comprises a proximal end for insertion into the body and a distal end; and the pouch and catheter are arranged such that during withdrawal of the catheter from the pouch, the distal end of the catheter is withdrawn from the pouch before the proximal end and the proximal end of the catheter passes through the channel.

Advantageously, the present invention provides for a packaged catheter that can be easily removed from its packaging and simultaneously lubricated ready for use. The join provides the advantage that as teh catheter is removed, it is drawn through the channel, in which the fluid is provided. The fluid is typically an activating liquid, such as water, which activates a hydrophilic coating to render it slippery, or may be a lubricant. Either way, drawing the catheter through the channel (in which it is preferably provided) ensures the catheter is properly lubricated. The channel of fluid increases the coating of fluid to the surface of the catheter as the fluid is held in a pool through which the catheter is forced to travel, rather than being potentially pulled out of the package without adequately wetting it. Moreover, the distal end, by which the catheter can be pulled out, should not pass through the pool of fluid, rendering it easy to handle.

Furthermore, the deliberate formation of a pool at the bottom means that fluid may be held within the package at all times, which makes the device more convenient to use and reduces the chance of spillages, as may occur where for example a reservoir of fluid is intended to be opened into the pouch, then the pouch shaken to distribute the fluid. The pouch can also be reduced in size due to the curve of the catheter when inside the pouch. The use of the join creating a channel also facilitates using less fluid as it is applied in a targeted manner to the part of the catheter that needs to be wetted, not distributed over the entire catheter. The invention also provides the advantage that the proximal end is the last part of the catheter to be wetted and therefore is least likely to dry up before it is introduced into the urethra. The volume of the channel is controlled by the position of the join permitting the use of flexible and soft pouch walls which are more user-friendly when carrying/using the pouch.

The fluid reservoir may be configured to release fluid into the pouch to form a pool of fluid in the channel. The volume of the fluid reservoir is preferably sufficient to completely fill the channel, i.e. with the pouch held with its base lowermost, the fluid may extend to at least the level of the bottom of the join, so that the fill level of the channel is 100% or more, for example at least 150%, at least 200% or even at least 300%. Alternatively, the fill level of the channel may be: at least 70%, at least 80%, at least 90%; or at least 95% the volume of the channel. Thus, the present invention provides for an assembly with a reservoir that is able to deliver fluid into the channel. The size of the reservoir is selected to ensure sufficient fluid is delivered and the catheter can be adequately lubricated on removal.

The fluid reservoir may be provided as a separate device, or incorporated into the pouch material. The fluid reservoir may comprise a deformable, frangible or burstable sachet. Thus the fluid reservoir is conveniently located within the pouch where it may directly release fluid into the channel formed by the join and the base of the peripheral seal. A deformable, frangible or burstable reservoir enhances the convenience for a user as fluid can be easily released without additional tools and before the pouch is unsealed, thus reducing the chances of accidental wetting of the user. A sachet in particular can be easily handled on an assembly line and introduced more simply than attempting to handle fluid, so as to it into the package during manufacture.

The catheter may be a urinary catheter. The catheter may be a male urinary catheter. The catheter may be an intermittent catheter. In one embodiment, the catheter is an intermittent male urinary catheter. Thus, the features of the present invention allow intermittent male urinary catheters to be provided in a compact packaging that also facilitates convenient and easy withdrawal of the catheter whilst simultaneously lubricating it prior to use.

The catheter may be arranged between the join and the internal base of the peripheral seal. The method may comprise arranging the catheter between the join and the internal base of the peripheral seal. The join may be separate from the base of the peripheral seal. The channel may be an open-ended channel. Thus, the catheter is held within the pouch in the open-ended channel formed by the join and the internal base of the peripheral seal. As such, when withdrawn, the catheter is drawn through the channel and is adequately lubricated.

The join and the peripheral seal may be independent. The join may be provided in a spot. The join is preferably provided by a weld, e.g. a spot weld. Such a weld may be provided after the catheter (and optionally the sachet) have been arranged between the walls, and can be achieved from outside of the pouch. Alternatively, the join may be provided by any of a: mechanical seal; heat seal; pressure seal; adhesive; solvent bond; ultraviolet bond; ultrasonic weld; laser weld; impulse weld; or friction weld. Similarly, the peripheral seal be provided by any one or more of a weld; mechanical seal; heat seal; pressure seal; adhesive; solvent bond; ultraviolet bond; ultrasonic weld; laser weld; impulse weld; or friction weld. At least part of the peripheral seal may be formed by a fold. In such embodiments, a single sheet may be folded, for example at the base or one of two sides, to form two opposing walls of the pouch. The join can be provided in a single location allowing the peripheral seal and join to be formed separately. Alternatively, the weld forming the join may be provided
at the same time as the peripheral seal. In some embodiments, the join and peripheral seal are a single continuous seal. The manufacturing process of the join can thus be very convenient and avoid major modification of a typical packaged catheter assembly production line.

The catheter may comprise a surface which is activated by the fluid in the channel. For example, the catheter may comprise a hydrophilic surface, formed for example by a hydrophilic coating. Where a hydrophilic surface is provided, the fluid may be polar or water-based. The catheter may comprise a hydrophobic surface, which is activated by the fluid in the channel. For example the catheter may comprise a hydrophobic coating. Where a hydrophobic coating is used, the fluid may be non-polar. Thus the catheter is easily lubricated by fluid in the channel and is easy and comfortable to use.

The pouch may comprise an interaction region defining a first point of contact for a user of the packaged catheter assembly. The interaction region may facilitate user access to the contents of the pouch. Actuation of the interaction region may create an opening in or near an upper edge of the peripheral seal. The catheter may be withdrawn through the opening. Thus, access to the pouch is provided at or near an upper edge of the pouch and as such, the fluid in the channel (at the bottom of the pouch) is retained in the pouch while the catheter is withdrawn reducing the risk of spilling fluid outside the pouch.

The interaction region may comprise a breakable region of the peripheral seal. The method may comprise forming a breakable region in the peripheral seal. The breakable region may comprise a tear-away region which may comprise a tear line defining a line along which the pouch may be torn to at least partially separate the tear-away region from the remainder of the pouch, thereby creating an opening in the peripheral seal. The tear-away region may comprise a tear start. The tear start may be provided at a first end of the tear away region. The tear start may comprise a notch, or area of weakness, in one or both of the walls of the pouch. The tear start may define the first part of the pouch to be torn. The tear-away region may comprise a tear stop. The tear stop may be provided at a second end of the tear-away region. The tear stop may act to prevent or reduce the likelihood of the tear-away region being completely separated from the remainder of the packaging. A tear line may be defined between the tear start and the tear stop. The tear line may be configured to preferentially tear. The tear line may comprise a weakened region of the pouch. The tear line may be formed by any suitable means, such as laser scoring. The tear-away region may be substantially triangular in shape having a sloped upper edge. Thus the breakable region provides a convenient and easy means to open the pouch with minimal effort and no additional equipment/tools.

The interaction region may comprise a sealing arrangement. The sealing arrangement may comprise one or more sealing members operable to provide a user-sealable seal between the two walls of the pouch, so as to close the pouch. In one embodiment, first and second sealing members are provided on an interior surface of the two walls of the pouch. The sealing arrangement may provide a watertight and/or fluid impermeable seal when closed. Thus the pouch can be resealed to ensure the fluid in the channel is not subsequently released from the pouch after use. This also allows the catheter to be repackaged back into the pouch after use and retained within it.

The sealing arrangement may come pre-sealed. In such embodiments, user access to the contents of the pouch may be provided by opening the sealing arrangement. The sealing arrangement may be resealable. The sealing arrangement may be any of: a zip-lock seal; hook and loop seal; hook and hook seal; or adhesive layer. Therefore, the sealing arrangement can be used to ensure the contents of the pouch remain inside until the sealing arrangement is opened, and the pouch can still be resealed after use. This can help if the breakable region requires force to be applied to the pouch to be opened, as the pouch may move or jerk in position while being opened causing fluid or the catheter to exit the pouch.

The interaction region may comprise a breakable region and a sealing arrangement. In certain embodiments, the interaction region comprises a tear-away region corresponding to the upper edge of the peripheral seal, and a resealable arrangement below the tear-away region.

The peripheral seal may have an upper edge. The peripheral seal may be substantially rectangular in shape with two lateral edges between the base and upper edge. The peripheral seal may have a height from the base to the upper edge of at least 10 cm, 15 cm, or 20 cm; and/or a height of no more than 10 cm, 15 cm, 20 cm or 25 cm, such as between 15 cm and 20 cm. The peripheral seal may have a width between the lateral edges of at least 5 cm, 10 cm; or 15 cm; and/or a width of no more than 10 cm or 15 cm, or 20 cm such as between 5 cm and 15 cm. The catheter may have a length of at least: 20 cm; 25 cm; 30 cm; 35 cm; 40 cm; 45 cm; or 50 cm, for example between 35 and 45cm. In one embodiment, the peripheral seal has a height 10-25 cm and a width of 6-11 cm, and the catheter has a length of at least 30 cm. Thus, the peripheral seal defines a "pocket-sized" pouch that can store a catheter within in a curved configuration to reduce the apparent size of the catheter and enable convenient and discreet storage.

The distal end of the catheter may be arranged closer to an upper edge of the peripheral seal than the base of the peripheral seal. The method may comprise arranging the distal end of the catheter closer to an upper edge of the peripheral seal than the base of the peripheral seal. The distal end of the catheter may be arranged in the upper half of the pouch, or the upper quarter of the pouch. The distal end of the catheter may be arranged above the join. The distal end of the catheter may be arranged above the fill level of the fluid. Thus, the distal end of the catheter is easy to access so that it may be withdrawn before the proximal end of the catheter as required by the invention. It is also positioned so as not to be pulled through the fluid, so will not be wetted and will remain easy to handle.

The distal end of the catheter may be provided with a handling sleeve, which may be arranged closer to an upper edge of the peripheral seal than the base of the peripheral seal. The method may comprise arranging a handling sleeve at the distal end of the catheter, at least partially closer to an upper edge of the peripheral seal than the base of the peripheral seal. At least part of the handling sleeve may be arranged in the upper half of the pouch or the upper quarter of the pouch. At least part of the handling sleeve may be arranged above the join. The entire handling sleeve may be above the join. The handling sleeve may be further from the join than the base of the pouch. At least part of the handling sleeve may be arranged above the fill level of the fluid. The entire handling sleeve may be arranged above the fill level of the fluid. Thus, the handling sleeve is easy to access so that it may be withdrawn before the proximal end of the catheter as required by the invention. It is also positioned so as not to be pulled through the fluid, so at least part of it will not be wetted and will remain easy to handle, providing a dry surface that can be gripped and used to manoeuvre the catheter.

Any one or more of the distal end of the catheter, the funnel, and the handling sleeve may be arranged centrally with respect to an opening formed in the pouch. The term arranged centrally may correspond to a position closer to a middle of the opening than an edge of the opening. The term arranged centrally may correspond to a position in a middle portion of the opening. The middle portion may be the middle 70%, 60%, 50%, 40%, or 30% of the opening. Any one or more of the distal end of the catheter, the funnel, and the handling sleeve may be arranged centrally within the pouch. Any one or more of the distal end of the catheter, the funnel, and the handling sleeve may be closer to a middle of the pouch then the left lateral edge and/or right lateral edge. As such, when the pouch is opened, the distal end/funnel/handling sleeve is more easily accessible as it is central and located at a point across the width of the pouch which opens widest.

The distal end of the catheter may be above the rest of the catheter within the pouch. The handling sleeve and/or funnel may extend across an opening formed in the pouch. The handling sleeve and/or funnel may extend from the distal end to a point adjacent to the left lateral edge or right lateral edge. The funnel may not be aligned parallel to the left lateral edge and/or right lateral edge. At least part of the handling sleeve may not be aligned parallel to the left lateral edge and/or right lateral edge. An end of the fluid reservoir may correspond to the position of the distal end of the catheter. The fluid reservoir may be adjacent to the right lateral edge or left lateral edge. Where the handling sleeve extends adjacent to one lateral edge (e.g. the left lateral edge or right lateral edge), the fluid reservoir may be adjacent to the other lateral edge (e.g. the right lateral edge or the left lateral edge). The distal end may be arranged adjacent to one lateral edge (e.g. the left lateral edge or right lateral edge) and the handling sleeve and/or funnel may extend to a point adjacent to the other lateral edge (e.g. the right lateral edge or the left lateral edge). Thus, the position of the distal end, funnel, handling sleeve and fluid reservoir protects the rest of the catheter from inadvertent direct contact with the user as they take the catheter out of the pouch. This reduces the risk of infection when using the catheter as it is less likely to become dirty before use.

The distal end of the catheter may be arranged closer to one (e.g. a left) lateral edge of the peripheral seal than another (e.g. a right) lateral edge of the peripheral seal. The method may comprise arranging the distal end of the catheter closer to one (e.g. a left) lateral edge of the peripheral seal than another (e.g. a right) lateral edge of the peripheral seal.

The join may be provided at a point equidistant from each lateral edge. Alternatively it may be offset. The method may comprise providing the join at a point equidistant from each lateral edge. Alternatively it may comprise proving the join in an offset lateral position. For example it may be arranged anywhere between 25% and 75% of the width of the pouch. The distance between the left and right lateral edges of the peripheral seal may be the width of the pouch. The distal end of the catheter may be provided at a distance from one left edge that is no more than 20%, 10%, 5% or 1% of the width of the pouch. Thus the join and distal end are provided in locations that make the catheter easy to withdraw and lubricate without spilling lubricant. The provision of a join that is equidistant between the edges ensures the channel volume is restricted and that the fluid level within it is optimised for lubricating the catheter.

In embodiments comprising an interaction region, actuation of the interaction region may preferentially first create an opening in a first (e.g. left) lateral edge of the peripheral seal. In such embodiments, the distal end of the catheter may be arranged closer to the first (e.g. left) lateral edge. In embodiments comprising a tear-away region and a tear start, the location of the tear start may correspond to a lateral edge of the peripheral seal. In such embodiments, the distal end of the catheter may be arranged closer to the lateral edge of the peripheral seal corresponding to the location of the tear start. Thus, the distal end is located next to the first part of the pouch to be opened and is easier to withdraw.

The join may be provided closer to the base of the peripheral seal than an upper edge of the peripheral seal. The distance between the base and the upper edge of the peripheral seal may define the height of the pouch. The distance between the join and base may be at least: 10%; 15%; 20%; 25%; 30%; 35%; 40% or 45% of the height of the pouch. The distance between the join and base may be less than: 50%; 45%; 40%; 35%; 30%; 25%; 20%; or 15% of the height of the pouch. In particular, the join can be arranged at between 20% and 30% of the height of the pouch. The join may be circular. The join may have a radius equivalent less than 15%; 10%; 5%; or 1% the height of the pouch. In a preferred embodiment, the join has a radius of 5% the height of the pouch. Thus, the join can be arranged to ensure the catheter is adequately lubricated and that it defines a channel of suitable size to allow easy withdrawal of the catheter without excessive bending of the catheter or excessive force to be applied.

In one embodiment, the join is provided at a point equidistant from each lateral edge of the pouch and at a distance from the base that is 20-30% of the height of the pouch.

The distal end of the catheter may be provided with a funnel arranged so as not to pass through the channel during withdrawal of the catheter. The handling sleeve may also be arranged so as not to pass through the channel during withdrawal of the catheter. The sleeve may be no more than: 25%; 20%; 15%; 10%; or 5% of the length of the catheter, but could be longer, even up to the full length of the catheter. Embodiments of the present invention provide a funnel and sleeve to allow easy handling of the catheter. These elements, or at least part of them, do not pass through the channel and so are not coated in fluid during withdrawal. The sleeve may not extend below the height of the join within the pouch. The sleeve may be further from the join than the base of the pouch. This reduces the likelihood that the sleeve is wetted during release of fluid into the channel from the fluid reservoir. As such, the catheter is more convenient to use as the fluid is only applied to the necessary parts of the catheter. It also facilitates using less fluid as it is applied in a targeted manner to the catheter.

The angle subtended by the ends of the catheter when curved in the pouch may be at least: 180; 210; 240; 270; 300; 330; or 360 degrees; it may even be at least 420; 480 or 540 degrees. It may be less than 720; 630 or 540 degrees. The catheter may be arranged within the pouch in a curled or coiled configuration. The join may be provided within an area defined by the curl or coil of the catheter. The peripheral seal may confine the catheter in its curved configuration. As such, the catheter is curved, curved or coiled to reduce the size of the pouch and ensure that long catheters can be conveniently and discreetly stored ready for use.

The catheter may be arranged such that during withdrawal at least: 30%; 40%; 50%; 60%; 70% or 80% of the length of the catheter passes through the channel. In one embodiment, 70-80% of the length of the catheter passes through the channel during withdrawal. Advantageously, only the part of the catheter intended to enter the body is lubricated and therefore fluid is not wasted and the catheter is easier to use.

The walls may be formed of any suitable material, such as film materials. The walls may be impermeable to fluids. The walls may be made of a plastics material. The walls may be made from any one or more of: polypropylene (PP), polyethylene terephthalate (PET), low density polyethylene (LDPE), polyethylene terephthalate polyester (MET PET) or orientated polypropylene (OPP), for example. The walls may comprise a laminate of two or more layers. Each wall may comprise an inner layer and an outer layer. Each wall may comprise inner and outer layers of polypropylene. Each wall may comprise an inner layer of orientated polypropylene and an outer layer of polypropylene. Each wall may comprise an inner layer of polypropylene and an outer layer of polyethylene, for example. The inner layer and outer layer may have the same thickness. In other embodiments, the inner layer and outer layer may have different thicknesses. The inner layer may have a smaller thickness than the outer layer. In embodiments, the inner layer may have a thickness of between 5-20 microns, or between 10-10 microns, or between 10-15 microns, and the outer layer may have a thickness of between 10-50 microns, or between 20-40 microns, or between 35-45 microns. In one example, the inner layer may have a thickness of approximately 12 microns and the outer layer may have a thickness of approximately 40 microns.

According to a third aspect of the present invention there is provided a method of wetting a catheter in a packaged catheter assembly according to claim 14.

The method of the third aspect may include a method of unpacking a packaged catheter assembly, the packaged catheter assembly comprising a pouch, a catheter contained within the pouch and a fluid reservoir, the method comprising releasing fluid from a reservoir to form a pool in a channel between a base of the pouch and a join, opening the pouch and pulling the catheter out by a distal end of the catheter, whereby as the distal end is pulled out, a proximal end of the catheter for insertion into the body is guided through the channel, so as to be wetted by the pool of fluid.

The packaged catheter assembly in the method of the third aspect may be a packaged catheter assembly as defined in general terms, or according to the first aspect set out above or one manufactured by the method of the second aspect set out above, optionally including any of the optional features set out above. Indeed it may comprise any of the optional features without necessarily including all the features set out in the first and second aspects above

The step of releasing fluid may comprise forming a pool in the channel that is at least 60%; 70%; 80% of the volume of the channel; in particular it may be at least 90%; or at least 100% the volume of the channel, even up to at least 150%, 200% or 300%. Thus the method provides for creation of a pool of fluid that is adequate to lubricate the catheter without excess lubricant being used.

The methods of using are not part of the claimed invention and are left for illustrative purposes.

The method of wetting of the third aspect may illustratively include a method of using a catheter comprising releasing fluid from a reservoir to form a pool in a channel between a base of the pouch and a join, opening the pouch and pulling the catheter out by a distal end of the catheter, whereby as the distal end is pulled out, a proximal end of the catheter for insertion into the body is guided through the channel, so as to be wetted by the pool of fluid; then introducing the catheter by its proximal end into the urethra. The urethra may be that of a human, especially a human male.

The method may further comprise releasing urine through a funnel at the distal end of the catheter. The method may further comprise removing the catheter. The method may further comprise coiling or folding the catheter up and introducing it back into the pouch for disposal. The method may further comprise sealing the used catheter in the pouch prior to disposal.

Again, the step of releasing fluid may comprise forming a pool in the channel that is at least 60%; 70%; 80% of the volume of the channel; in particular at least 90%; or 100% and even up to at least 150%, 200% or 300% of the volume of the channel. Thus the method provides for creation of a pool of fluid that is adequate to lubricate the catheter without excess lubricant being used.

The pouch may comprise two opaque walls. The window may be provided in one of the opaque walls. The window may be spaced from at least one edge of the pouch or the wall it is provided in. The window may be spaced from all edges of the pouch. The window may be less than: 25%; 20%; 15%; 10% or 5% the total area of the pouch and/or the wall it is provided in. The window may correspond to the size and/or shape of the fluid reservoir. The window may be a smaller size than the fluid reservoir. In one embodiment, the window is the same shape but a smaller size than the fluid reservoir. Thus, in embodiments of the invention, the window advantageously corresponds to the size, shape and position of the fluid reservoir and is provided in a discrete manner, separated from the edges of the pouch and only provided in a section of the pouch's wall.

The window may correspond to the position of the fluid reservoir. The window may correspond to the position of an indicator region of the pouch. The indicator region may correspond to a wetting region, which may extend from the base of the pouch to a level at which sufficient fluid has escaped from the pouch to wet the catheter. The indicator region may be configured to indicate successful release of fluid from the reservoir. The indicator region may comprise a fill-level marker indicating sufficient fluid is in the wetting region. Thus the window also provides a visual guide to ensure fluid released from the reservoir has been delivered to a specific area of the pouch (e.g. a channel) or that it has successfully lubricated the catheter.

The pouch may be substantially rectangular in shape with two lateral edges, a base, and an upper edge. The height of the pouch may be defined as the distance between the base and upper edge. The width of the pouch may be defined as the distance between the lateral edges of the pouch. The window may span at least: 20%, 30%; 40%; 50%; 60%; 70%; 80% or 90% the height of the pouch. In one embodiment, the window spans at least 30-40% of the height of the pouch and the window is between 15-20% of the width of the pouch. Thus, the fluid reservoir is easily visible as the window is aligned with it, but simultaneously, the area of the window is minimised to ensure the contents of the pouch remain generally not visible, on account of the fact that but for the window, the pouch is opaque.

Those skilled in the art will understand that where the pouch is described as opaque, but includes a transparent or translucent window, it is predominantly opaque, but for the window; for example, entirely opaque apart from the window. It will also be understood that more than one window may be provided, for example a window may be provided on each of two otherwise predominantly or entirely opaque opposing walls, or indeed two windows may be provided on one (or more) otherwise opaque wall.

This is to be contrasted with known catheter packages formed from (only) two opposing walls: one opaque paper wall and one transparent plastic film wall; these cannot be considered to be opaque but for a window, because the transparent film wall makes up the entire second wall of the packaging. They cannot be considered to have an opaque wall with a with a transparent or translucent window in it.

The catheter may be hidden by the opaque walls of the pouch. The catheter may be hidden by the fluid reservoir. This is particularly advantageous from the point of view of discretion -the catheter is likely to appear to observers as some kind of medical device, even if they are not aware of its precise nature; hiding it, either by ensuring that it is arranged out of register with the window, or by hiding it behind another object, for example the fluid reservoir (but optionally a dedicated sheet material, for example) obscures this.

Notably, the fluid reservoir (or other obscuring object) may be opaque, and may have a colour that corresponds to, or contrasts with that of the walls of the pouch. For example, the reservoir (or other obscuring object) may be in the same colour, but a different shade to that of the pouch, or where the opaque pouch has multiple colours, may be a shade of one of those colours. A grey or silver is considered particularly preferable for the reservoir (or other obscuring object) from the point of view of discretion.

The window may be patterned; i.e. at least part of the window may be provided with a pattern thereon, such as a pattern formed by opaque regions, such as spots, stripes or the like. The pattern may provide a graduated appearance to the window. The pattern may provide a graduated appearance to one or more of the edges the window. The pattern may blend the window into the surrounding packaging of the pouch. Thus, the window is less obvious to the untrained eye and is therefore the packaged catheter assembly is more discreet for the user to carry with them.

A retaining seal may be provided between the two walls to restrict the movement of the fluid reservoir (which may of course be a sachet as defined above) in the pouch. The method may comprise the step of forming a retaining seal between the two walls to restrict the movement of the fluid reservoir in the pouch. The retaining seal may be provided outside the curvature of the catheter. The retaining seal may be closer to an upper edge of a peripheral seal of the pouch than a base of the pouch. The retaining seal may extend from an upper edge of a peripheral seal of the pouch.

A portion of window may correspond to location that is lower than the join in the pouch. Thus where a channel defined by the join is used to lubricate the catheter, the fluid level in the channel can be visually verified using the window.

The join may act as the retaining seal to restrict movement of the fluid reservoir. In such embodiments, the fluid reservoir may be arranged between the join and a lateral edge of the peripheral seal. In some embodiments, the fluid reservoir may be arranged adjacent to a first lateral edge of the peripheral seal and the join may be provided closer to the first lateral edge than the other (second) lateral edge of the peripheral seal. Thus, the retaining seal ensures the fluid reservoir does not move inside the pouch and that it is always visible through the window (and in a preferred embodiment always hiding the catheter).

### Detailed Description of the Invention

In order that the invention may be more clearly understood one or more embodiments thereof will now be described, by way of example only, with reference to the accompanying drawings, of which:
- Figure 1: is an exploded view of a first embodiment of a packaged catheter assembly;
- Figure 2: is a front view of the sealed assembly of Figure 1 prior to use;
- Figure 3: is a front view of the packaged catheter assembly of Figures 1 and 2 where fluid has been released into the pouch;
- Figure 4: is a front view of the packaged catheter assembly of Figures 1-3 where the package has been opened;
- Figure 5: is a front view of the packaged catheter assembly of Figures 1-4 where the catheter has been part withdrawn from the pouch;
- Figure 6: is a front view of the packaged catheter assembly of Figures 1-5 where the catheter has been almost fully withdrawn from the pouch;
- Figure 7: is a front view of a used packaged catheter assembly of Figures 1-6;
- Figure 8: is a front view of a second embodiment of a packaged catheter assembly; and,
- Figure 9: is a cut-away front view of a third embodiment of a packaged catheter assembly.

In the figures, as is conventional, broken lines show hidden features.

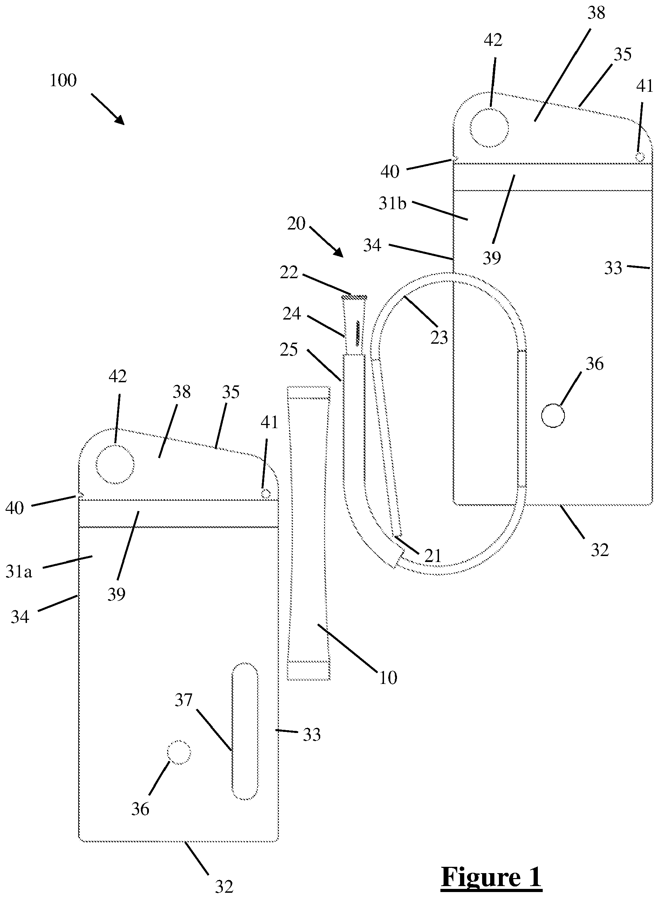

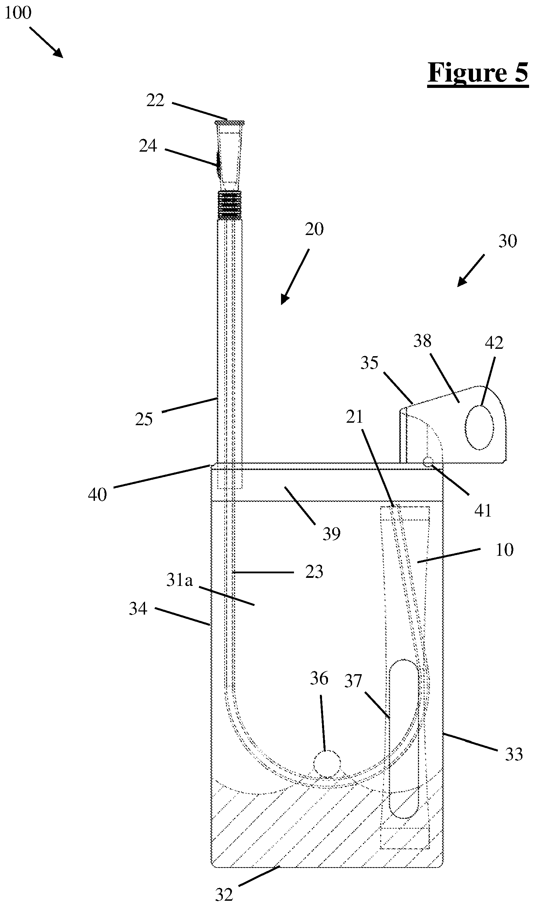

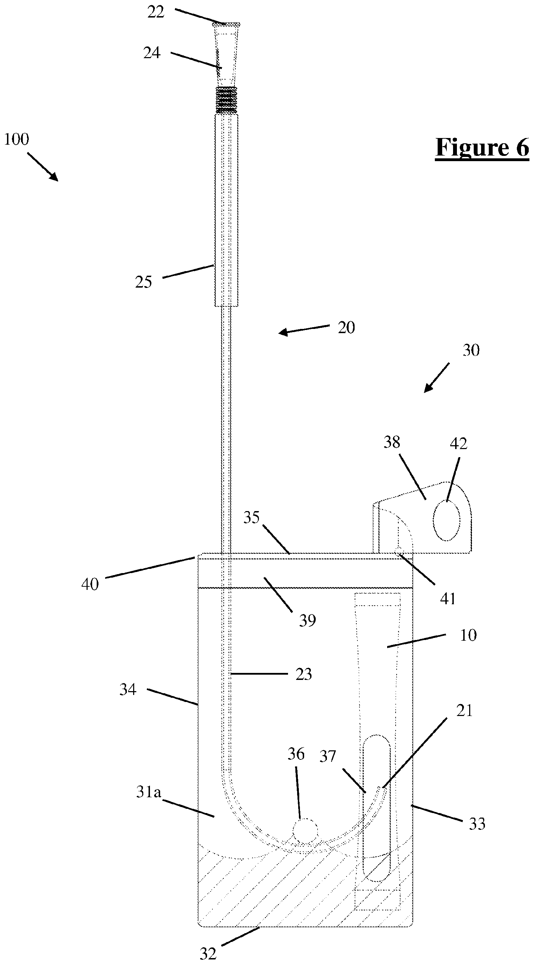

Referring to Figures 1-7, a first embodiment of a packaged catheter assembly 100 is shown.

The assembly 100 comprises a fluid reservoir 10, a male intermittent single-use urinary catheter 20, and a pouch 30. The pouch 30 comprises a front opaque wall 31a, a rear opaque wall 31b opaque wall, and a peripheral seal joining the periphery of the walls 31a, 31b together to form the pouch. The peripheral seal comprises a base 32, a right lateral edge 33, a left lateral edge 34, and an upper edge 35. The left lateral edge 34 and right lateral edge 33 being defined as the left and right sides of the pouch when viewing the pouch 30 with the rear wall 31b behind the front wall 31a, the base 32 at the bottom of the pouch 30 and the upper edge 35 at the top of the pouch 30. The peripheral seal thus defines a pouch 30 that is generally rectangular and suitably has a width between the lateral edges of between 60-110 mm, for example about 100mm, e.g. 95 mm, and a height from the base 32 to the upper edge 35 of between 100 to 250 mm, for example about 200 mm, e.g. 197 mm. The base 32 defines the bottom of the pouch in use, and the upper edge 35 the top. However, in other embodiments alternative shapes and sizes could be conceived, such as an elliptical or circular pouch.

The pouch also comprises a join 36 between the two walls 31a, 31b. The join 36 is a circular weld provided at point equidistant between the lateral edges 33, 34 of the peripherals seal and at a distance above the base 32 extending from 45 to 55mm. The join 36 may have a radius between 1% and 10% the height of the pouch, for example 5%. In other embodiments the join may be provided in different shapes and sizes, at different locations, or could potentially even be integrated into a continuous seal with the peripheral seal.

The pouch also comprises a window 37 in one of the walls, which allows a user to see inside the pouch. In this particular embodiment, the window 37 is transparent elongate and generally rectangular with a length between 20-50% the height of the pouch 30, for example 33%, and width between 5-25% the width of the pouch 30, for example 12.5%. In this example, the window 37 has semi-circular ends, but in other embodiments they may be any suitable shape, such as flat ends or irregularly shaped ends. The window 37 is arranged parallel to the right lateral edge 33, and a gap is provided between the edge of the window 37 and right lateral edge 33 equivalent to between 5- 20% the width of the pouch, for example 10%. The bottom of the window 37 is separated from the base of the peripheral seal 32 by a distance equal to between 5-20% the height of the pouch, for example 10%. In other embodiments, translucent windows may be used and additionally alternative shapes, sizes and positions or angles of the window are contemplated (for example say at an angle of up to 20 degrees with respect to the edges.

In this particular embodiment, each wall 31a, 31b comprises a plurality of layers of foil; in one specific embodiment a 97 micron 2-layer foil of 12 micron PET and 60 micron LLDPE. In other embodiments, the walls 31a, 31b may only comprise a single layer, and different plastics materials may be used interchangeably.

As mentioned above, the walls 31a, 31b are opaque. This may be achieved by forming each wall 31a, 31b from one or more opaque layers of material or printing an opaque pattern onto one of the layers. The window 37 in the front wall 31a, may be formed by laminating an opaque layer of material onto a transparent wall, wherein the opaque layer has an aperture corresponding to the window 37. Alternatively, a transparent wall may be printed on to block light from passing through the wall, rendering it opaque in all places except for the location of the window 37. Further alternatives may also be contemplated and achieved, for example, providing an aperture corresponding to the window 37 in an opaque wall, and bonding a transparent layer so as to cover the aperture and achieve a continuous front wall 31a with a window 37, or starting with a multi-layer foil with an opaque layer and a transparent layer and removing part of the opaque layer of foil to form the transparent window.

In this particular embodiment, the pouch also comprises an interaction region. The interaction region forms the top part of the pouch 30 and spans between the right lateral edge 33 and left lateral edge 34. The interaction region is used to provide access to the pouch through/near to the upper edge 35 . In this embodiment, the interaction region comprises a tapered tear away region 38 and a sealing arrangement 39. The tear away region 38 comprises a tear start 40 on the left lateral edge, about 2cm below at the point the upper edge 35 and left lateral edge 34 meet, and a tear stop 41 at a point in the pouch 30 about 1cm below where the upper 35 and right lateral 33 edges meet. The distance from the tear stop 41 and the right lateral edge 33 being between 1-10% the width of the pouch, for example 5%. The tear start 40 comprises a notch to provide an area of weakness in the walls 31a, 31b allowing the tear away region 38 to be separated from the pouch 30 by tearing the walls apart from the tear start 40 to the tear stop 41. Thus the tear is provided beneath the location of the upper edge 35 and thus the pouch 30 is opened provided access to its contents. The tear stop 41 comprises a small aperture in the walls 31a, 31b to prevent further tearing of the pouch 30 and separation of the tear away region 38 from the pouch 30. In order to provide the tapered shape, the tear away region 38 extends upwards on the left side approximately 50% more than it does on the right side. The left side of the tear away region 38 comprises a circular aperture 42 sized to allow a finger to pass through and grip the tear away region 38. To ensure the pouch is fully sealed, the front wall 31a and rear wall 31b are sealed to one another around the periphery of the tear stop 41 and circular aperture 42. In other embodiments, other seals such as a zip-lock arrangement may be used to provide access to the pouch, and of course, the sizes and shapes set out above are exemplary.

In this embodiment, the sealing arrangement 39 extends between the left lateral edge 34 and right lateral edge 33 of the peripheral seal just below the tear away region 38. The sealing arrangement 39 comprises first and second sealing members (not shown) provided on an interior surface of the walls 31a, 31b of the pouch 30. In this embodiment, the sealing members comprise corresponding sealing grooves and ridges to allow the sealing members to be press-fit together to form a water-tight seal and pulled apart again if necessary. In other embodiments, a single adhesive sealing member may be used, or a sealing member may be provided on the outside of the pouch to allow the pouch to be folded or rolled and sealed accordingly and of course, alternative sealing mechanisms like a hook-and-loop or hook-and-hook seal or zip-lock could be used.

In this embodiment, the fluid reservoir 10 is an elongate rectangular burstable sachet of water, but in other embodiments any suitable reservoir may be used. The fluid reservoir 10 has a height equal to between 60-90% the height of the pouch 30, for example 80%, and a width between 5-25% the width of the pouch 30, for example 15%. In this embodiment, the fluid contained within the reservoir 10 is water. In other embodiments, many other types of reservoir would be suitable including different shapes and sizes of reservoir, or non-burstable reservoirs and other external sources of fluid. In addition, other fluids could be used, for example lubricants, gels, oils or the like.

In this embodiment, the catheter 20 comprises a proximal end 21 for insertion into the body, a distal end 22, and a flexible tube 23 connecting the two ends 21, 22. The distal end 22 comprises a funnel 24 to guide urine from the bladder out of the tube 23 in use. A handling sleeve 25 is also provided around the tube 23 adjacent to the distal end 22. In this embodiment, the catheter has a length of between 30-50 cm, for example 40 cm, and the handling sleeve is between 10 and 30% the length of the catheter, for example 20-25%, e.g. 11 cm. In this embodiment, the catheter 20 is provided with a hydrophilic coating, this is activated when it contacts the fluid held in the reservoir 10. In other embodiments, any suitable catheter length or type may be used.

The catheter 20 is arranged within the pouch 30 in a curved and coiled configuration, with the distal end 21 adjacent to the inner left lateral edge 33 near the upper 35 edge of the pouch 30, just beneath the sealing arrangement 39. The catheter 20 extends down the inside of the pouch 30 into a broadly elliptical anti-clockwise coil (as viewed with left lateral edge 34 on the left, and upper edge 35 at the top). The coil having a single turn around the inner perimeter of the left lateral edge 34, base 23, and right lateral edge 33 of the pouch, with the proximal end 21 of the catheter 20 resting on the inside of the catheter's coil, adjacent to the left lateral edge 34 and base 32 of the pouch. Notably, the majority of the handling sleeve 25 is arranged above the join 36, but as the catheter curves and extends from left to right, the catheter tube 23 runs underneath the join, in a channel defined by the join and the internal base of the peripheral seal, then runs upwards to the side of the join, before returning over the top of the join and finally extending downwardly such that the proximal end 21 is lower than and on the left side of the join 36. In short, the catheter 20 is coiled (or curled) around the join 36 (albeit not tightly coiled). In other embodiments, the position of the catheter 20 may be different, in particular, the catheter 20 may not coil or curl but may simply curve within the pouch 30. On the other hand, embodiments of the invention may have a catheter 20 that has multiple turns of coil.

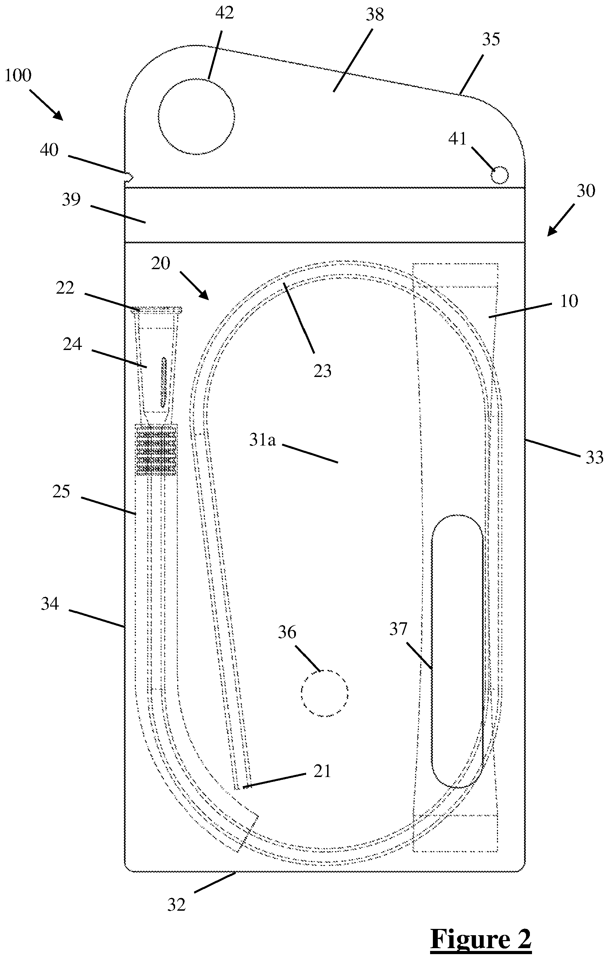

Referring to Figure 2, the pouch 30 is formed by first providing the two walls 31a, 31b which are to define the pouch 30 itself, and the front wall 31a comprising the transparent window 37 as described above and produced by one of the techniques described above. In this embodiment, the walls 31a, 31b are sized to correspond to the final shape of the pouch 30, however in other embodiments, the walls may extend outside the pouch 30 as required.

In this embodiment, the base 32, right lateral edge 33 and left lateral edge 34 are the first to be formed by welding the two walls 31a, 31b together, leaving the pouch 30 with an open upper edge 35. The catheter 20 is then arranged within the pouch 30 in a curved and coiled configuration, as set out above

In this embodiment, the fluid reservoir 10 is also placed within the pouch 30 at a position corresponding to the window 37. The catheter 20 is therefore hidden in the pouch 30, as the reservoir 10 is placed over the catheter 20 so that the reservoir 10 is visible through the window 37 and the reservoir 10 obstructs any view of the catheter 20 through the window 37. In other embodiments, the reservoir 10 may not need to be placed over the catheter 20 as the position of the catheter 20 in the pouch 30 may not correspond to the window 37.

In this embodiment, the first and second sealing elements (not shown) are then attached to the inside of the walls 31a, 31b just above the coiled catheter 20, and the peripheral seal is completed by forming its upper edge 35 by welding the walls 31a, 31b together along the upper edge 35. This seals the catheter 20 and reservoir 10 within the pouch. In other embodiments, the sealing elements may be provided to the walls prior to any other assembly of the pouch 30, or sealing of the peripheral seal.

In this embodiment, the join 36 is then formed by welding the walls 31a, 31b together. Because the catheter 20 is coiled around the inner perimeter of the pouch 30, the join 36 is located within the coil of the catheter 20. Therefore, part of the catheter tube 23 passes between the join 36 and the base 32 of the pouch. In other embodiments, the join 36 may be provided before the peripheral seal, or at any point of the assembly process.

Finally, in this embodiment, the tear-start 40, tear-stop 41, and aperture 42 may be formed by cutting out the appropriate sections of the pouch 30. Use of a hot punch can simultaneously bond the walls 31a, 31b around the periphery of the cut-out regions to ensure the pouch 30 remains fully sealed.

As will be appreciated, the above is an example and in other embodiments the pouch 30 may be formed in different ways with the steps performed in different orders. For example, the bottom could be open and the catheter and water reservoir introduced from the bottom before the base seal is provided. Or, for example, in an industrially applied process, two webs of wall material may have one half of the sealing arrangement applied to (an interior surface of) each of them, the catheter 20 and reservoir 10 may be arranged between opposing interior surfaces of the webs of wall material, then potentially in a single action, a hot die may join and form the walls 31a, 31b by forming the peripheral seals 32, 33, 34, 35, simultaneously forming the tear start 40, forming the join 36 and punching out and sealing the aperture 42 and tear stop 41 in the tear away region 38. Furthermore, while the peripheral seal and join 36 have been described as formed by a weld, any suitable means of joining the walls could be used, for example, an adhesive may be used.

Referring to Figures 2-7, this first embodiment of a packaged catheter assembly 100 may be unpackaged as described below.

First, the fluid reservoir 10 is located within the pouch 30 with the assistance of the window 37, though which the reservoir 10 can be seen. Fluid is then released from the reservoir 10 into the pouch 30. In this embodiment, the reservoir 10 is a burstable sachet and fluid may be released from it by applying manual pressure to the reservoir 10. The window 37 provides a visual reference to show when the reservoir 10 has been successfully burst and fluid released into the pouch 30. In particular, it can be seen that sufficient fluid has been released to fill the channel defined by the weld 36 and the internal base 32 of the peripheral seal. In other embodiments, the fluid may be released from other means.

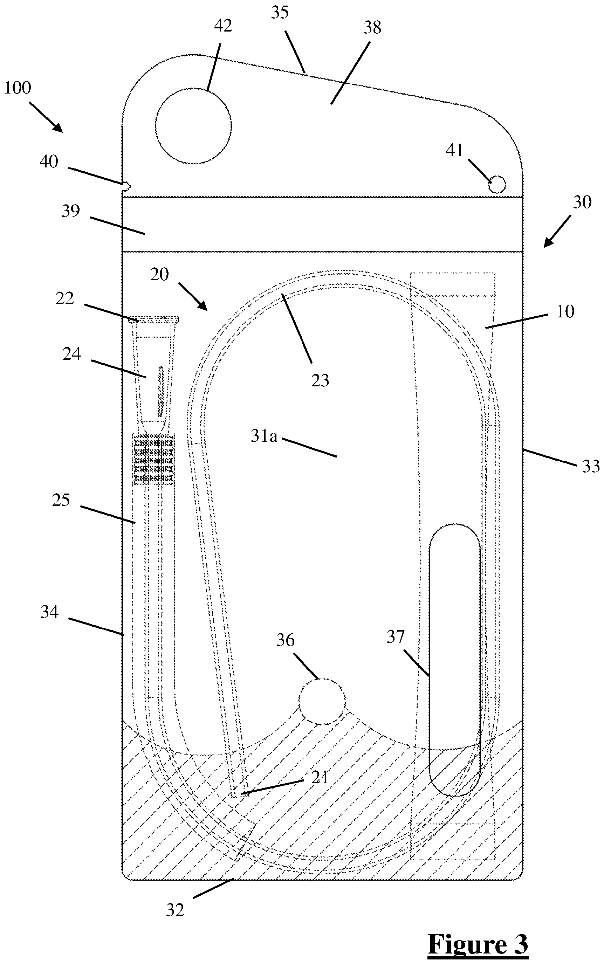

Referring to Figures 3-7, the fluid released from the reservoir 10 collects in a pool at the bottom of the pouch 30, as shown by the hashing. In this embodiment, the fluid fills the pouch 30 up to a fill level corresponding to the join 36. As such, in this embodiment, the join 36 and base 32 define a channel that is completely full of fluid. However, in other embodiments, the fluid may only partially fill the channel, for example filling the channel between 60-95%, preferably at least 90%, or may fill the pouch above the level of the join 36.

The released fluid is also visible through the window 37, as the base of the window 37 corresponds to a location on the pouch 30 that is slightly lower than the join 36. In other embodiments, the window may comprise an indicator region that shows the presence of fluid in the pouch. For example, a fluid-sensitive colour changing strip such that direct visualisation of the fluid is not required. A fill-level marker could also be provided to show that sufficient fluid has been released.

In this embodiment, the contents of the pouch 30 are then accessed by tearing the top of the pouch 30 using the tear away region 38. The aperture 42 is grasped in one hand, and the rest of the pouch 30 in another, and the tear away region 38 is torn from the tear start 40 to the tear stop 41. Thus, the pouch is torn between the left lateral edge 34 and right lateral edge 33 at a location below the upper edge 35, as such, an opening is formed in the pouch 30 that may be used to access its contents. Advantageously, the tear away region 38 is not fully separated from the pouch 30 which reduces the number of separate parts and makes the pouch 30 easier to handle. Other embodiments may feature other ways to access the pouch for example a zip-lock seal.

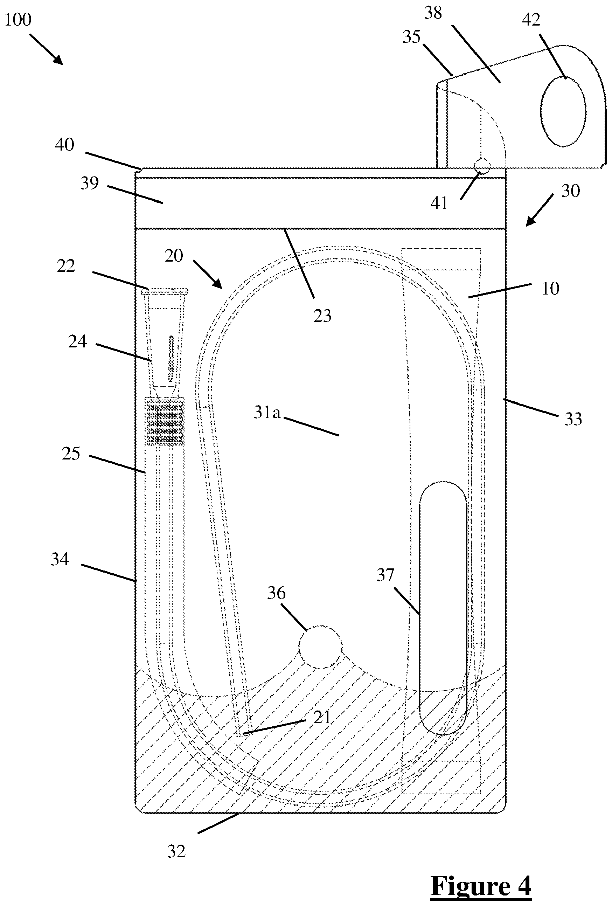

Referring to Figures 4-6, the catheter 20 may then be withdrawn from the pouch 30. As described above, in this embodiment the catheter 20 is coiled in the pouch 30, and the distal end 21 is provided adjacent the upper edge 35 of the pouch 30. As such, the distal end 21 is above the fill line of the fluid released into the pouch 30 and is not wetted by the fluid. The distal end 21 therefore provides a dry and easy to access point which may be used to withdraw the catheter 20 from the pouch 30. In this embodiment, the majority of the handling sleeve 25, i.e. at least 50% and preferably between 60-100%, for example 70% of the sleeve 25 is also above the fill line and is not wetted. Thus, the sleeve 25 also provides a convenient dry surface to handle the catheter 20.

In this embodiment, the distal end 21 is pulled upwards through the opening in the upper edge 35 of the pouch 30. As the distal end 21 is pulled out of the pouch 30, the catheter tube 23 is uncoiled and also pulled upwards towards the upper edge 35 of the pouch 30. However, as the join 36 is arranged within the coil of the catheter 20, the tube 23 is prevented from moving directly upwards by the join 36. The join 36 therefore causes the tube 23 to be withdrawn from the pouch 30 along a path that includes passing through the channel defined by the join 36 and base 32. As such, the entire remainder of the tube 23 to the proximal tip 21 passes though the pool of fluid in the channel and the hydrophilic coating of the catheter 20 is activated by the fluid. Furthermore, the proximal end 21 of the catheter 20 is the final part of the catheter 20 to pass through the channel and be withdrawn from the pouch 30. Thus, the catheter 20 is activated by the fluid as it is withdrawn and the withdrawal is convenient and easy as only a dry part of the catheter 20 needs to be touched during withdrawal. As the catheter 20 passes through a pool of fluid it is more completely wetted than would be the case for a fluid held within another medium such as a foam. In other embodiments, the catheter 20 need not be withdrawn directly upwards but may be pulled out to sideways from the pouch, for example from an opening provided at the side of the pouch, but, of course, above the weld, so as to reduce the prospect of spillage. Further the catheter 20 may not directly touch the join 36 and may instead be prevented from moving upwards by the convergence of the walls 31a, 31b as they approach the join 36.

Once the catheter is fully withdrawn, the catheter may be used by inserting the proximal end 21 into the urethra until urine begins to flow through the catheter 20. The (largely dry) handling sleeve 25 assists in the insertion. The urine may then be directed out of the catheter 20 by the funnel 24.

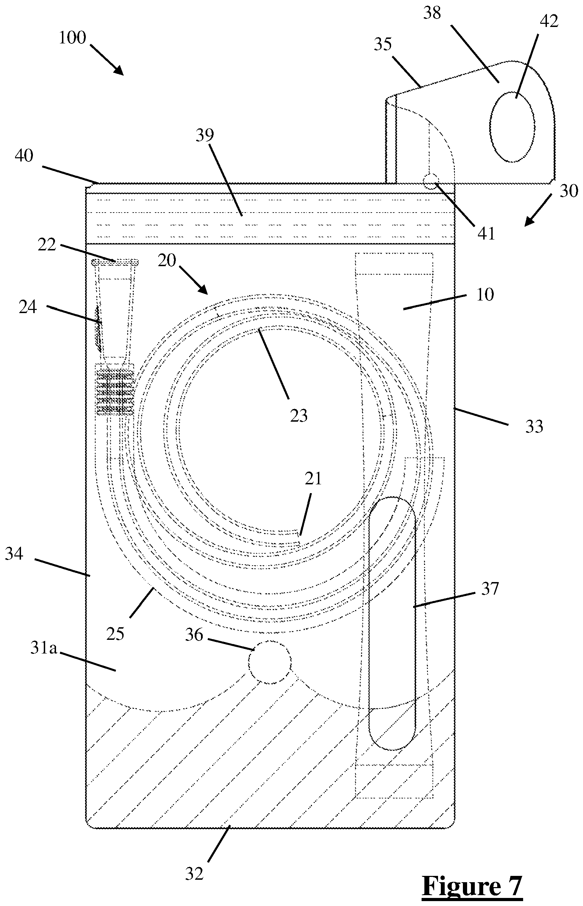

Referring to Figure 7, in this embodiment, the catheter 20 may be placed back inside the pouch 30 in a coiled configuration after use. The handling sleeve 25 and funnel 24 provide for dry surfaces that can be used to re-coil the catheter 20 and place it into the pouch 30. The sealing arrangement 39 can then be used to reseal the pouch 30 between the left lateral edge 34 and right lateral edge 33 by pressing together the two corresponding sealing elements (not shown). This provides for a more convenient disposal of the catheter 20 after use and ensures the fluid released into the pouch 30 does not subsequently leak out of the pouch 30. In other embodiments, alternative sealing arrangements may be used, such as an external flap with adhesive that allows the top of the pouch 30 to be rolled or folded and sealed. Alternatively, no sealing arrangement may be used where the pouch 30 is intended to be disposed of immediately after use.

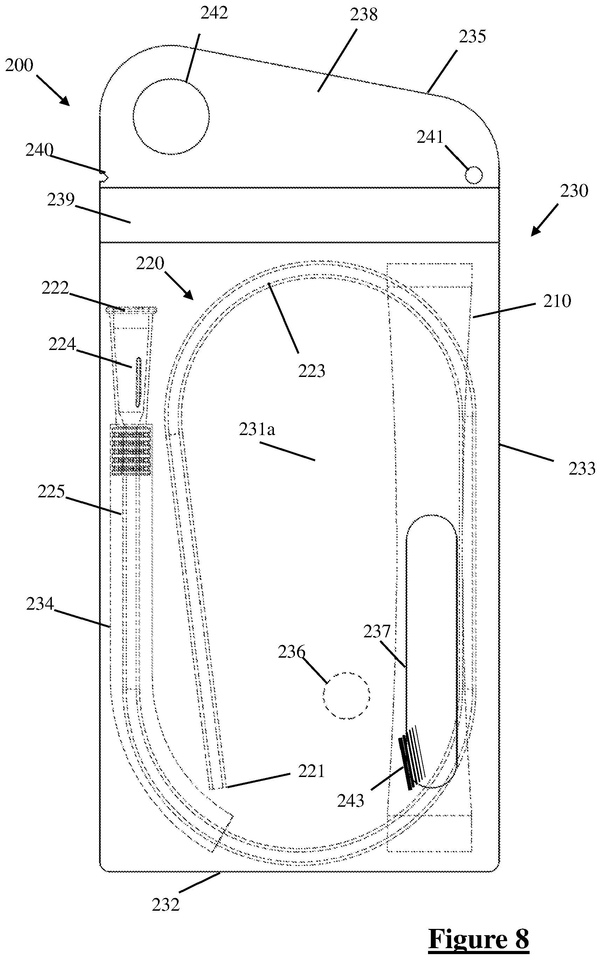

Referring to Figure 8, a second embodiment of a packaged catheter assembly 200 is shown. The second embodiment shares many of the features of the first embodiment described above, and so only differences in the features are described below, and like numerals are used to denote like features.

In this embodiment, the join 236 is provided a location offset from the midpoint between the right lateral edge 233 and left lateral edge 234. In particular, the join is provided at a distance from the right lateral edge 234 that is 20-45% the width of the pouch 230, for example, 35%. The join 236 provides a second function as a retaining seal to restrict the movement of the reservoir 210 in the pouch 230, sandwiching the reservoir 210 between the join 236 and the right lateral edge 234. The join 236 therefore ensures the window 237 corresponds to the position of the reservoir 210 regardless of the movement of the pouch 230 as it is carried around by a user, ensuring both that the catheter 223 remains hidden and that the reservoir 210 can be identified easily to release its fluid. In other embodiments, the function of the retaining seal may be provided by a separate seal between the walls 231a, 231b. For example, a separate seal extending downwards from the sealing arrangement 239, or up from the base 232.

In this embodiment, the front wall 231a also comprises a pattern 243 that corresponds to the edge of the window 237. The pattern 243 is only shown on part of the window, but may of course extend over the entirety of it and comprises a graduated appearance that softens the boundary between the transparent window 237 and the rest of the opaque front wall 231a. In this embodiment, the pattern is a series of evenly spaced opaque lines with graduated thicknesses, getting thinner as they approach the centre of the window 237. As such, the pattern 243 makes the window 237 less visually obvious and therefore makes the pouch 230 more discreet. In other embodiments, the pattern 243 could be any suitable pattern, for example opaque circles of graduated size, and may be complemented by or integrate with surface patterns or colour applied to the reservoir 210.

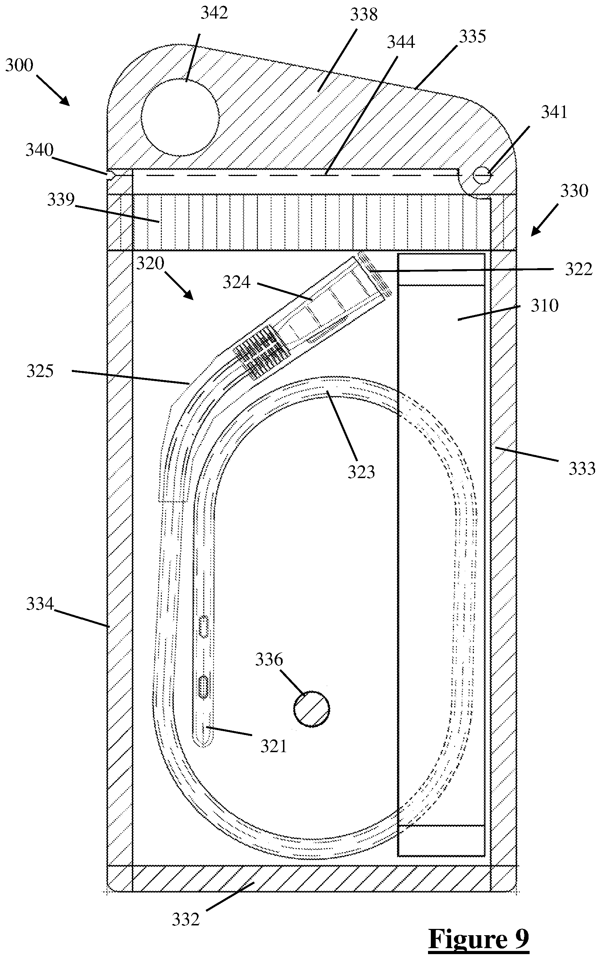

Referring to Figure 9, a third embodiment of a packaged catheter assembly 300 is shown. The third embodiment shares many of the features of the first embodiment described above, and so only differences in the features are described below, and like numerals are used to denote like features. Figure 9 shows a cut-away front view of the assembly 300 and as such, front wall 331a and window 337 are not shown. Diagonal hashing shows the locations in which the front wall 331a is bonded to the rear wall 331b, for example at the left lateral edge 334, base 322, right lateral edge 333, upper edge 335, tear off region 338 and join 336.

In this embodiment, the tear away region 338 comprises a tear line 343 spanning between the tear start 340 and tear stop 341. The tear line 344 is a line of weakness that preferentially tears to allow the tear away region 338. This makes it easier to remove the tear away region 338 in a consistent manner. In this embodiment, the tear line 344 is formed by weakening of the pouch 330 by laser scoring but in other embodiments other techniques could be used such as thinning of the pouch 330 material along the tear line 344.

In this embodiment, the sealing arrangement 339 comprises a zip-lock seal to allow the user to reseal the pouch 330 closed. The location in which the sealing arrangement 339 can be used to seal the front wall 331a and rear wall 331b together is denoted in Figure 9 by vertical hashing. This corresponds to a strip of the pouch 330 just below the tear line 344.

In this embodiment, the catheter 320 is arranged within the pouch 330 in a similar manner to the first embodiment in that it is coiled. However, in this embodiment, the distal end 322 of the catheter 320 and funnel 324 are arranged centrally with respect to the opening formed by the tear away region 338. The distal end 322 and funnel 324 are approximately equidistant from the left lateral edge 334 and right lateral edge 333 rather than closer to either lateral edge 333, 334. As such, when the pouch 330 is opened, the distal end 322 including funnel 324 and handling sleeve 324 are more easily accessible as it is central and located at a point across the width of the pouch 330 which opens widest. In addition, as the catheter 320 is coiled, the handling sleeve 325 extends from the distal end 322 to a positing adjacent the left lateral edge 334. Thus, together with the fluid reservoir 310 which is positioned adjacent to the right lateral edge 33, the distal end 322, handling sleeve 325 and fluid reservoir 310 protect the rest of the catheter 323 from inadvertent direct contact with the user as they withdraw the catheter 320 from the pouch 330. This reduces the risk of infection when using the catheter 320 as it is less likely to become dirty before use.

In this embodiment, the sleeve 325 is configured so that it does not extend below the height of the join 336 within the pouch 330, in particular, the sleeve 325 is further from the join 336 than the base 332 of the pouch 330. This reduces the likelihood that the sleeve 325 is wetted during release of fluid into the channel from the fluid reservoir 310. As such, the sleeve 325 is easier for the user to handle.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment.

The one or more embodiments are described aboe by way of example only. Many variations are possible without departing from the scope of protection afforded by the appended claims.

## Claims

1. A packaged catheter assembly (100) comprising a catheter (20), a fluid reservoir (10), and an opaque pouch (30) containing the fluid reservoir and catheter, wherein the pouch comprises a transparent or translucent window (37) **characterised in that** the catheter in the pouch is hidden.

2. The assembly as claimed in claim 1 wherein the pouch comprises two opaque walls, and the window is provided in one of the opaque walls.

3. The assembly as claimed in claim 1 or claim 2 wherein the catheter is a male urinary catheter.

4. The assembly as claimed in any preceding claim wherein the catheter is an intermittent catheter.

5. The assembly as claimed in any preceding claim wherein the window corresponds to the position of the fluid reservoir in the pouch.

6. The assembly as claimed in any preceding claim wherein the window corresponds to the position of an indicator region of the pouch, wherein the indicator region is configured to indicate successful release of fluid from the reservoir.

7. The assembly as claimed in any preceding claim wherein the catheter is hidden by the fluid reservoir.

8. The assembly as claimed in any preceding claim wherein the catheter is hidden by the pouch.

9. The assembly as claimed in any preceding claim wherein the window comprises a pattern that provides a graduated appearance to one or more of the edges the window.

10. The assembly as claimed in any preceding claim wherein the pouch comprises two opaque walls, and the window is provided in one of the opaque walls and the fluid reservoir is a different colour or different shade of colour to the pouch.

11. The assembly as claimed in any preceding claim wherein the pouch comprises a retaining seal configured to restrict the movement of the fluid reservoir within the pouch.

12. A method of forming a packaged catheter assembly (100), the method comprising: providing two opaque walls to define a pouch (30); providing a fluid reservoir (10) within the pouch; arranging a catheter (20) within the pouch; forming a peripheral seal between the walls to seal the catheter and fluid reservoir within the pouch; and providing a transparent or translucent window (37) in at least one wall of the pouch **characterised in that** the catheter is hidden in the pouch..

13. The method as claimed in claim 12 wherein the packaged catheter assembly is the packaged catheter assembly according to any of claims 1 to 23.

14. A method of wetting a catheter (20) in a packaged catheter assembly (100), the method comprising releasing fluid from a reservoir (10) in a pouch (30), and inspecting the interior of the pouch through a transparent or translucent window (37) in an opaque wall of the pouch, either to identify the reservoir to release the fluid or to confirm that the reservoir has released fluid into the pouch or both, and removing the catheter from the pouch **characterised in that** the catheter is hidden in the pouch.

15. The method of as claimed in claim 14 wherein the packaged catheter assembly is the assembly of any of claims 1 to 11and/or the packaged catheter assembly is formed according to the method of claim 12 or 13.

## Patentansprüche

1. Verpackte Katheteranordnung (100), umfassend einen Katheter (20), ein Fluidreservoir (10) und einen undurchsichtigen Beutel (30), der das Fluidreservoir und den Katheter enthält, wobei der Beutel ein durchsichtiges oder durchscheinendes Fenster (37) umfasst, **dadurch gekennzeichnet, dass** der Katheter in dem Beutel verdeckt ist.

2. Anordnung nach Anspruch 1, wobei der Beutel zwei undurchsichtige Wände aufweist und das Fenster in einer der undurchsichtigen Wände angeordnet ist.

3. Anordnung nach Anspruch 1 oder Anspruch 2, wobei der Katheter ein männlicher Harnkatheter ist.

4. Anordnung nach einem der vorhergehenden Ansprüche, wobei der Katheter ein intermittierender Katheter ist.

5. Anordnung nach einem der vorhergehenden Ansprüche, wobei das Fenster der Position des Fluidreservoirs in dem Beutel entspricht.

6. Anordnung nach einem der vorhergehenden Ansprüche, wobei das Fenster der Position eines Indikatorbereichs des Beutels entspricht, wobei der Indikatorbereich dazu ausgebildet ist, die erfolgreiche Freigabe von Fluid aus dem Reservoir anzuzeigen.

7. Anordnung nach einem der vorhergehenden Ansprüche, wobei der Katheter durch das Fluidreservoir verdeckt ist.

8. Anordnung nach einem der vorhergehenden Ansprüche, wobei der Katheter durch den Beutel verdeckt ist.

9. Anordnung nach einem der vorhergehenden Ansprüche, wobei das Fenster ein Muster aufweist, das einem oder mehreren der Ränder des Fensters ein abgestuftes Aussehen verleiht.

10. Anordnung nach einem der vorhergehenden Ansprüche, wobei der Beutel zwei undurchsichtige Wände aufweist und das Fenster in einer der undurchsichtigen Wände angeordnet ist und das Fluidreservoir eine andere Farbe oder einen anderen Farbton als der Beutel aufweist.

11. Anordnung nach einem der vorhergehenden Ansprüche, wobei der Beutel eine Rückhaltedichtung aufweist, die dazu ausgebildet ist, die Bewegung des Fluidreservoirs innerhalb des Beutels zu begrenzen.

12. Verfahren zum Ausbilden einer verpackten Katheteranordnung (100), das Verfahren umfassend: Anordnen von zwei undurchsichtigen Wänden, um einen Beutel (30) zu definieren; Anordnen eines Fluidreservoirs (10) innerhalb des Beutels; Anordnen eines Katheters (20) innerhalb des Beutels; Ausbilden einer Umfangsdichtung zwischen den Wänden, um den Katheter und das Fluidreservoir innerhalb des Beutels abzudichten; und Anordnen eines durchsichtigen oder durchscheinenden Fensters (37) in zumindest einer Wand des Beutels, **dadurch gekennzeichnet, dass** der Katheter in dem Beutel verdeckt ist.

13. Verfahren nach Anspruch 12, wobei die verpackte Katheteranordnung die verpackte Katheteranordnung nach einem der Ansprüche 1 bis 23 ist.

14. Verfahren zum Befeuchten eines Katheters (20) in einer verpackten Katheteranordnung (100), das Verfahren umfassend Freisetzen von Fluid aus einem Reservoir (10) in einem Beutel (30) und Untersuchen des Inneren des Beutels durch ein durchsichtiges oder durchscheinendes Fenster (37) in einer undurchsichtigen Wand des Beutels, entweder um das Reservoir zu identifizieren, um das Fluid freizusetzen, oder um zu bestätigen, dass das Reservoir Fluid in den Beutel freigesetzt hat, oder beides, und Entfernen des Katheters aus dem Beutel, **dadurch gekennzeichnet, dass** der Katheter in dem Beutel verdeckt ist.

15. Verfahren nach Anspruch 14, wobei die verpackte Katheteranordnung die Anordnung nach einem der Ansprüche 1 bis 11 ist und/oder die verpackte Katheteranordnung gemäß dem Verfahren nach Anspruch 12 oder 13 ausgebildet ist.

## Revendications

1. Ensemble (100) à cathéter emballé comprenant un cathéter (20), un réservoir (10) de fluide et un sachet (30) opaque contenant le réservoir de fluide et le cathéter, dans lequel le sachet comprend une fenêtre (37) transparente ou translucide, **caractérisé en ce que** le cathéter dans le sachet est caché.

2. Ensemble suivant la revendication 1, dans lequel le sachet comprend deux parois opaques et la fenêtre est prévue dans l'une des parois opaques.

3. Ensemble suivant la revendication 1 ou la revendication 2, dans lequel le cathéter est un cathéter urinaire masculin.

4. Ensemble suivant l'une quelconque des revendications précédentes, dans lequel le cathéter est un cathéter intermittent.

5. Ensemble suivant l'une quelconque des revendications précédentes, dans lequel la fenêtre correspond à la position du réservoir de fluide dans le sachet.

6. Ensemble suivant l'une quelconque des revendications précédentes, dans lequel la fenêtre correspond à la position d'une région d'indicateur du sachet, dans lequel la région d'indicateur est configurée pour indiquer une libération de fluide du réservoir couronnée de succès.

7. Ensemble suivant l'une quelconque des revendications précédentes, dans lequel le cathéter est caché par le réservoir de fluide.

8. Ensemble suivant l'une quelconque des revendications précédentes, dans lequel le cathéter est caché par le sachet.

9. Ensemble suivant l'une quelconque des revendications précédentes, dans lequel la fenêtre comprend un motif, qui procure un aspect gradué à l'un ou à plusieurs des bords de la fenêtre.

10. Ensemble suivant l'une quelconque des revendications précédentes, dans lequel le sachet comprend deux parois opaques, et la fenêtre est prévue dans l'une des parois opaques et le réservoir de fluide est d'une couleur différente ou d'une teinte de couleur différente du sachet.

11. Ensemble suivant l'une quelconque des revendications précédentes, dans lequel le sachet comprend un joint de retenue configuré pour restreindre le déplacement du réservoir de fluide dans le sachet.

12. Procédé de formation d'un ensemble (100) à cathéter emballé, le procédé comprenant : se procurer deux parois opaques pour définir un sachet (30) ; mettre un réservoir (10) de fluide dans le sachet ; mettre un cathéter (20) dans le sachet ; former un joint périphérique entre les parois pour rendre le cathéter et le réservoir de fluide étanches dans le sachet, et prévoir une fenêtre (37) transparente ou translucide dans au moins une paroi du sachet, **caractérisé en ce que** le cathéter est caché dans le sachet.

13. Procédé suivant la revendication 12, dans lequel l'ensemble à cathéter emballé est l'ensemble à cathéter emballé suivant l'une quelconque des revendications 1 à 11.

14. Procédé de mouillage d'un cathéter (20) dans un ensemble (100) à cathéter emballé, le procédé comprenant libérer du fluide d'un réservoir (10) dans un sachet (30) et inspecter l'intérieur du sachet à travers une fenêtre (37) transparente ou translucide dans une paroi opaque du sachet, soit pour identifier le réservoir pour libérer le fluide, soit pour confirmer que le réservoir a libéré du fluide dans le sachet ou les deux et retirer le cathéter du sachet, **caractérisé en ce que** le cathéter est caché dans le sachet.

15. Procédé suivant la revendication 14, dans lequel l'ensemble à cathéter emballé est l'ensemble suivant l'une quelconque des revendications 1 à 11 et/ou l'ensemble à cathéter emballé est formé suivant le procédé de la revendication 12 ou 13.
